# EUROPEAN PATENT SPECIFICATION

(11) **EP 4 312 906 B1**
(45) Date of publication and mention of the grant of the patent: **09.12.2026**
(21) Application number: 22715346.7
(22) Date of filing: 29.03.2022
(51) Int. Cl.: A61F 5/44, A61F 5/445, A61F 13/34

(54) **ABSORBENT INSERT DEVICES FOR COLLECTING LIQUID AND SEMI-LIQUID INTESTINAL WASTE INSIDE THE INTESTINE OF A HUMAN**
SAUGFÄHIGE EINSETZVORRICHTUNGEN ZUM SAMMELN VON FLÜSSIGEM UND HALBFLÜSSIGEM DARMABFALL IM DARM EINES MENSCHEN
DISPOSITIFS D'INSERT ABSORBANT POUR COLLECTER DES DÉCHETS INTESTINAUX LIQUIDES ET SEMI-LIQUIDES À L'INTÉRIEUR DE L'INTESTIN D'UN ÊTRE HUMAIN

(30) Priority: 30.03.2021 DK PA202100331
(43) Date of publication of application: 07.02.2024
(73) Proprietor: Ampa Medical ApS, 1159 København K (DK)
(72) Inventor: PARSLOV, Marie Filippson, 2300 København S (DK); AMMITZBØLL, Cecilie, 2720 Vanløse (DK)
(74) Representative: Zacco Denmark A/S
(86) International application number: PCT/DK2022/050066
(87) International publication number: WO 2022/207051

(56) References cited:
- CN-B- 102 348 443
- DE-B3- 102007 062 133
- US-A- 2 603 213
- US-A- 4 328 804
- US-A- 4 979 947
- US-A- 4 981 465
- US-A1- 2013 030 392

## Description

### Technical Field

The invention relates to absorbent insert devices for collecting liquid and semi-liquid intestinal waste inside the intestine of a human. In particular, the present disclosure relates to absorbent insert devices for insertion through an ileostomy of a subject.

### Background art

An ileostomy is an artificial intestinal opening where the large intestine (colon) and sometimes part of the small intestine is surgically removed due to illness or trauma and the remaining part of the small intestine (jejunum or ileum) is redirected through the abdominal wall and sutured to the skin, constituting the ileostomy. Hereafter the intestinal waste is emptied through the ileostomy. Ileostomies do not have a sphincter to control voluntary excretion of intestinal waste and therefore the ileostomy is incontinent with continuous and uncontrollable excretion of intestinal waste. For the majority of people with ileostomy the intestinal waste is liquid and the volume is 500-1000 ml/day. The intestinal waste from an ileostomy is collected in a stoma bag attached to a person's stomach by an adhesive plate. The stoma bag was invented in the 1950's and has since then been used to collect the intestinal waste from an ileostomy. However, the stoma bag has considerable disadvantages.

First of all, the changed body image that comes with having a bag with intestinal waste hanging from the abdomen is a psychological strain for most people. Secondly, since a person with an ileostomy lacks the ability to hold back intestinal waste and intestinal gases, and the excretion of intestinal waste and intestinal gases into the stoma bag cause considerably noise and a characteristic smell during bowel movement, the user is often considerably socially handicapped - even when stoma bags are used properly. The adhesively attached stoma bags moreover have the disadvantage that the weight of the more or less filled bag causes discomfort and may result in the bag becoming involuntarily detached from the user's skin, resulting in leakage of intestinal waste. In addition, natural folding in the skin surrounding the stoma can cause difficulties in keeping the adhesive plate tight from leakage. The constant worry of leakage is a mental burden that almost all users of stoma bags live with. Moreover, users of stoma bags often get skin complications requiring medical treatment, due to skin contact with intestinal waste and the aggressive adhesive. Finally, physical exercise is complicated by the external storage of intestinal waste in a bag. Upon pushing, squeezing or clamping the bag there is a risk of leakage. In general, stoma bags require a protruding stoma, i.e. a stoma that protrudes a couple of centimetres from the skin. A protruding stoma works as a kind of faucet, such that the intestinal waste is guided into the bag, while minimizing the contact with the surrounding skin. However, this is not always successfully achieved and this leads to skin ulcerations. Furthermore, the protruding stoma may add to the mental burden of the user of the stoma bag.

People with ileostomies demand alternative solutions to the stoma bag. So far, there are no functional alternatives on the market. Existing solutions that are designed for withholding solid stool in a colostomy (stoma of the large intestine) are not suited for use in an ileostomy because of the large volumes of liquid content that is produced in the ileum. This makes it difficult to seal the ileum properly.

In general, tampon-like closures have the drawback that they typically have a small absorption capacity. If used in the intestine, this may result in intestinal waste not being able to be absorbed by the closure and thereby leak or accumulate behind the closure, which leads to an increased pressure on the intestinal wall. The increased pressure can result in dilation of the intestine, or it can result in the closure being pushed out of the intestine, both of which are undesirable situations. A further challenge with a tampon-like closure is that the diameter of the stoma is smaller than the elastic diameter of the intestine, whereby there is a risk of squeezing the closure upon pulling it out through the stoma, whereby some of the liquid intestinal waste is spilled. For these reasons, no tampon-like solution has ever been commercialized for usage in ostomies.

A further challenge is how to provide an alternative closure that can absorb liquid faeces in the rectum, which is particularly relevant for subjects suffering from anal incontinence. Anal incontinence can be caused by sphincter dysfunction or conditions causing chronic diarrhoea. Intestinal wastes from an incontinent normal anal opening are sometimes collected with diaper-like devices, but it is physically uncomfortable and mentally stigmatizing, which is why it is often preferred to close an incontinent anus with an actual closure. Closures for anal openings are on the market but their absorption capacity is very limited, making them ideal for people with solid faeces while people with liquid faeces often experience leakage and are dependent on diaper-like solutions.

US 4 981 465 A describes disposable closure means for an artificial ostomy opening or an incontinent natural anus.

US 4 328 804 A describes a hygienic tampon and tampon applicator.

CN 102 348 443 B describes a lubricated tampon having invertible flaps for facilitated insertion and removal.

US 2 603 213 A describes a bandage designed for post-operative use following surgery in the anus or vagina.

US 2013/030392 A1 describes an applicator member and flexible retention web device.

US 4 979 947 A describes an encapsulated expandible continence device.

DE102007062133B3 describes a device for temporary ileo- or urostomy closure.

### Summary

In the following, an ileostomy refers to any stoma from the small intestine that is from the jejunum or ileum. The device disclosed herein is hence placed in the jejunal or ileal part of the small intestine, which is jointly referred to as the ileum. The device is further suitable for usage in urostomies and in rectum for people with anal incontinence.

There is a need of a closure that is able to absorb liquid in the intestine while forming a closure hereof, and with the ability to retain the majority of that liquid, while being withdrawn from the intestine. In particular, there is a need of a closure that is suited for placement in the small intestine (ileum) and form a closure hereof, and with the ability to retain the majority of that liquid, while being withdrawn from the ileum.

The present disclosure solves the above-mentioned problems by providing an absorbent insert device for insertion through a stoma, such as an ileostomy, of a subject, such as a human being as defined in claim 1 of the appended claims.

The device is configured with a material and a configuration that enables it to enter the stoma opening, expand radially and longitudinally to a configuration tailored to the dimension of the intestine while absorbing intestinal waste and retain the intestinal waste while being withdrawn through the stoma, hereby the device is configured to absorb and/or collect intestinal waste inside the intestine, such as the ileum, of the subject, and comprises an expandable elongated body having an initial length and comprising an absorbent material, and withdrawal means attached to the elongated body, wherein the expandable elongated body is configured to expand radially upon insertion into the intestine through the stoma to form a leak-tight closure of the stoma. This means that the overall initial diameter (as seen in a cross section through the longitudinal axis of the device) of the device prior to insertion is smaller than the overall diameter (as seen in a cross section through the longitudinal axis of the device) of the device after it has been inserted into the stoma and released. This can be done by using various compression means, such as utilising an insertion sleeve or applicator surrounding the absorbent material prior to insertion or by inclosing the absorbent material in foil or plastic that is dissolved when the device is inserted in the stoma. The absorbent material hereby undergoes a change from a compressed state in which it has a size that makes it able to enter the stoma to a non-compressed state (i.e. the material expands), such that it makes a tight closure of the stoma e.g. by being placed against the inside walls of the ilium or by the intestinal mucosa folding tightly around the device. By leak-tight is meant that substantial all liquid is maintained within the stoma and not passing through. If small amounts are present on the outer surface of the stoma, a patch can be applied to overcome this problem.

The disclosed insert device is further configured to expand longitudinally, e.g. during absorption of the intestinal waste, thereby increasing the length of the elongated body inside the intestine. The longitudinal expansion of the device may also occur naturally by the device, subsequent to insertion of the device into the intestine. Both the radial and/or longitudinal expansion can be achieved by relieving the device from compressing forces keeping the device in a compressed state. As an example, said forces may be provided by a coating, which dissolves after a while subsequent to insertion, whereby the device is free to expand once the coating is dissolved. Alternatively, the insert device is compressed in an applicator and inserted into the body canal, or inserted with a guiding catheter that is placed in the centre of the absorbing insert device. Preferably, the device conforms approximately to the diameter of the stoma when pulled out of the intestine by means of the withdrawal means.

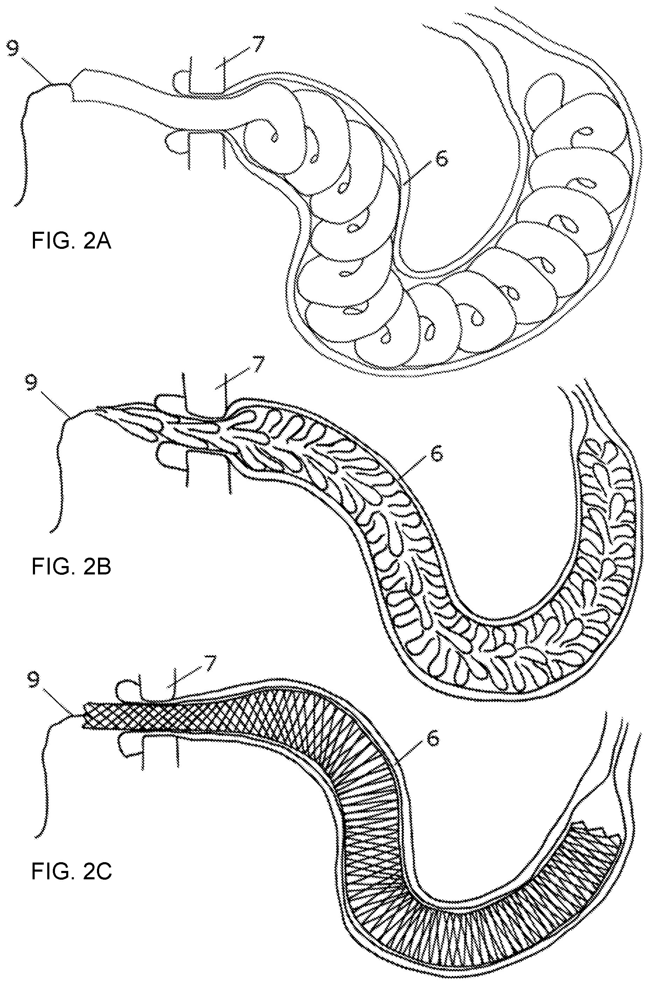

The present disclosure addresses the above-mentioned challenges (of collecting and retaining liquid intestinal waste in the intestine) by providing an absorbent insert device, which is configured for expanding radially and longitudinally to form a closure of the stoma of interest (e.g. the ileostomy or urostomy) and further configured such that it can be withdrawn from the stoma while retaining a majority of the liquid content, ideally all of the liquid content. This is achieved by the presently disclosed insert device, which can be provided in many different embodiments (cf. figures 2A-2F). Here it can be seen that all the different embodiments provide devices where the device is able to be pulled out through the stoma in such a way that the diameter of the cross section of the device at the stoma when pulled out, substantially corresponds to the diameter of the stoma. In this way, the absorbent material is not squeezed, and the liquid inside the absorbent material is maintained herein.

In particular, the presently disclosed device solves the problem of how to withdraw an absorbent closure holding an amount of absorbed liquid content from a body canal out through a stoma or natural intestinal opening. This is problematic as natural or artificial intestinal openings has a smaller diameter than the body canal, which makes it challenging to withdraw an absorbent closure through the opening, without squeezing the closure and thereby spilling some of the absorbed intestinal waste inside the body canal, e.g. intestine. The applicant has solved these challenges by providing an insert device that can be described by (at least) two diameters: a first diameter that should preferably be equal to or less than the diameter of the stoma of the subject, and a second diameter that is preferably approximately equal to the diameter of the body canal, wherein the insert device is intended to be placed. Prior to insertion through the stoma, the absorbent insert device is compressed to a size such that it can be inserted through the stoma. After insertion and removal of the compressing force (e.g. provided by a coating or an insertion applicator), the insert device preferably expands radially to largely conform to the diameter of the body canal behind the stoma, wherein it is placed, in order to form a closure of said body canal. Hence, subsequent to insertion, the insert device preferably expands to obtain the second diameter. However, even after expansion and absorbance of intestinal waste, the insert device is still able to be withdrawn from the body canal out through the stoma while retaining (ideally) all of the absorbed liquid content, or most of the liquid content. This ability is preferably achieved by configuring the insert device such that it conforms to the diameter of the stoma, e.g. achieved by the first diameter, when pulled out of the body canal by means of the withdrawal means.

The present disclosure further relates to an absorbent insert ileostomy device for insertion through an ileostomy of a human being, the device being configured to absorb intestinal waste inside the ileum of the human being and to form a leak-tight closure of the ileostomy, the device comprising:
an expandable elongated body comprising an absorbent material;
withdrawal means attached to the elongated body; and
one or more compression means;

wherein the compression means is configured to maintain the expandable elongated body in a compressed state, having a first diameter, prior to insertion;
wherein the expandable elongated body is configured to expand radially to a non-compressed state, having a second diameter, when released from the compression means upon insertion into the ileum through the ileostomy; and
wherein the first diameter is equal to or less than the diameter of the ileostomy of the human being and the second diameter is equal to or larger than the diameter of the ileum of the human being, wherein the insert device has a diameter of 1-3 cm and a length of 5-20 cm in the compressed state, and wherein the absorbent material may be configured such that the longitudinal axis is increased by at least 100% from the compressed state to the non-compressed state,
wherein the length of the expandable elongated body in the non-compressed state (13) along the longitudinal axis is at least 15 cm, and wherein the second diameter is between 2 and 5 cm.

The present disclosure further relates to an example of a kit of parts comprising: the absorbent insert device as described herein and a receptacle configured for receiving and containing the absorbent insert device after said device has been withdrawn from the subject. Furthermore, a separate bandage or cap may form part of the kit, said bandage/cap configured to keep the mucosal surface of the stoma moist. The bandage/cap should preferably be worn continuously and a filter, such as a charcoal filter, is preferably integrated to avoid unwanted odours. The receptacle may be an integrated part of the bandage/cap or alternatively an attachment system is integrated in the bandage/cap to attach the receptacle upon extraction of the absorbent insert device. The kit of parts may further comprise a sensor embedded or attached to the absorbent insert device. The sensor is preferably configured to detect if the absorbent insert device has been wetted (e.g. due to absorption of liquid), and/or detect microbial agents and/or configured to provide biochemical information such as pH level, level of electrolytes, and/or presence of blood. The receptacle may be provided with an adhesive in one end, such that it can be removably attached to the subject. The receptacle is preferably shaped largely as a cylinder, the cylinder being flexible and expandable in the longitudinal direction. This may be achieved by providing the cylinder with bellows, e.g. similar to the bellows of an accordion, such that the cylinder can be collapsed/folded in the longitudinal direction and subsequently be unfolded/expanded in the same direction. Hence, the receptacle can (similar to the insert device) be associated with a compressed stated and an expanded state. In another embodiment, the receptacle is an elongated sleeve configured to receive and store the presently disclosed insert device after it has been used. The elongated sleeve can preferably be collapsed and subsequently unfolded, whereby the insert device is withdrawn into the sleeve concurrent with the unfolding of the sleeve. The receptacle is preferably attached to the subject while in the compressed/folded state. The receptacle should be placed such that it covers the stoma, where the insert device is located behind. After the absorbent insert device has absorbed intestinal waste from the subject, the insert device can be withdrawn into the receptacle e.g. by pulling the withdrawal means. Once the user pulls both of these, the receptacle is configured to expand to a length, such that it is able to contain the entire absorbent insert device. Preferably, the receptacle is made from a biodegradable material such as a biodegradable plastic film. The advantage of the receptacle is that it provides a barrier to the absorbent insert device, which contains intestinal waste. Hence, the receptacle minimizes the risk of contamination from the insert device, when handling/removing the insert device after absorbance of intestinal waste. Both the absorbent insert device and the receptacle are preferably disposable such that they can be disposed together after use. Ideally, they can be flushed out in the toilet, in case both the absorbent device and the receptacle are made of a biodegradable material. The receptacle, as disclosed herein, may also be provided in itself for use with absorbent insert devices.

The present disclosure further relates to an example of a method for collecting intestinal waste from the intestine, such as the ileum, of a subject, the method comprising the steps of: inserting the absorbent insert device as described herein through the stoma of a subject, thereby placing the device inside the intestine, such as the ileum, of the subject; letting the absorbent material absorb intestinal waste from the subject; and withdrawing the absorbent insert device from the subject by use of withdrawal means attached to the absorbent insert device. The method may further comprise the step of withdrawing the absorbent insert device into a receptacle. The receptacle may preferably comprise a biodegradable material such as a biodegradable plastic film. Preferably, the absorbent insert device, upon pulling it out through the ileostomy, conforms to the diameter of the ileostomy while retaining a majority of the absorbed intestinal waste in the absorbent insert device. Preferably, the majority corresponds to at least 75 % of the absorbed intestinal waste, more preferably at least 85 %, and most preferably at least 95 %.

The present disclosure further relates to an example of use of the absorbent insert device as described herein to absorb intestinal waste inside the intestine, such as the ileum, of a subject such as a human being.

The method and use of the absorbent insert device as disclosed herein has the benefit that it completely avoids the use of a stoma bag to collect intestinal waste. Stoma bags are often associated with discomfort and psychological strain of the wearer. The presently disclosed absorbent insert device is configured for use inside the intestine, e.g. the ileum, of the wearer. Hence, it is not visible from the outside and thereby presents a more discreet solution to the user. Furthermore, the presently disclosed insert device does not require a protruding stoma, as is the case when using a stoma bag. Hence, the surgical procedure in relation to artificially created stomas can be changed in the future, such that the stoma is level with the skin of the person.

All of the disclosed embodiments of the absorbent insert device as described herein can be used in the disclosed method for collecting intestinal waste from the intestine of a subject. Furthermore, all of the disclosed embodiments can form part of the kit of parts comprising the absorbent insert device and the receptacle.

### Brief description of the drawings

Fig. 1A shows the absorbent insert device according to an embodiment, wherein the elongated body is curled around a longitudinal axis of the body to form a helix comprising a plurality of turns.
Fig. 1B shows the absorbent insert device according to an embodiment, wherein the elongated body comprises a plurality of flexible protrusions extending radially from the body, such that the elongated body resembles a brush.
Fig. 1C shows the absorbent insert device according to an embodiment, wherein the elongated body of the insert device is provided as a flexible tube, which is preferably partly or entirely filled with the absorbent material.
Fig. 1D shows the absorbent insert device according to an embodiment, wherein the elongated body of the insert device is formed from a sheet or a strip, which is rolled into an elongated conical shape.
Fig. 1E shows the absorbent insert device according to an embodiment, wherein the elongated body of the insert device is formed from a tube having a closed end, wherein the tube is folded one or more times inside itself.
Fig. 1F shows the absorbent insert device according to an embodiment, wherein the elongated body of the insert device is formed from a plurality of cylinders, each cylinder comprising an absorbent material.
Figures 2A-2F shows a non-compressed state of the insert device according to the corresponding (compressed) embodiment of fig. 1A-1F.
Figures 3A-3F shows cross-sections of the embodiments shown in figures 1A-1F. These cross-sections correspond to the compressed state of the insert device.
Figures 4A-4F shows cross-sections of the embodiments shown in figures 2A-2F. These cross-sections correspond to the non-compressed state of the insert device.
Fig. 5A shows the absorbent insert device according to an embodiment, wherein the elongated body is formed from a sheet that is folded to form a wave-like pattern of crests and troughs.
Fig. 5B shows the absorbent insert device according to an embodiment, wherein the elongated body constitutes a cylinder that is folded one or more times.

### Detailed description

The present disclosure relates to an absorbent insert device suitable for insertion through a stoma of a subject such as a human being. A stoma should be understood herein as any opening in the body, natural or artificial. An example of a natural stoma is the anus, and an example of an artificial stoma is an ileostomy. Behind the body opening is located a body canal such as the rectum, the colon, or the ileum. In one or more embodiments, the stoma is an opening on the abdomen that can be connected to either the digestive or urinary system to allow waste (urine or faeces) to be diverted out of the body. In one or more embodiments, the stoma is an ileostomy.

The absorbent insert device is configured to be placed inside such a body canal and form a closure of the body canal. In particular, the absorbent insert device as disclosed herein is suited for insertion through an ileostomy and for placement inside the ileum of the subject. The absorbent insert device comprises an absorbent material for absorbing liquids, in particular intestinal waste of the subject.

An ileostomy is an opening in the belly (abdominal wall) that is made during surgery. It is usually needed because a problem is causing the ileum to not work properly, or a disease is affecting that part of the colon and it needs to be removed. The end of the ileum (the lowest part of the small intestine) is brought through this opening to form a stoma. An ileostomy may only be needed for a short time (temporary), maybe for 3 to 6 months, because that part of the colon needs time to rest and heal from a problem or disease. But sometimes a disease, such as cancer, is more serious and an ileostomy may be needed for the rest of a person's life (permanent). Besides usage in an ileostomy, the present invention can be used for other related purposes, such as preventing leakage from urostomies, jejunostomies and from rectum in anal incontinence.

Preferably, the absorbent insert device is held in a compressed state prior to insertion through the stoma of the subject, said compressed state ensuring that the insert device can be inserted through the stoma. The insert device, in the compressed state, may have a cylindrical shape, preferably slightly conical with a smaller diameter at one end, said diameter being equal to or less than the diameter of the stoma of the subject. The insert device **is** compressed both longitudinally and radially. The insert device has a diameter of approximately 1-3 cm and a length of approximately 5-20 cm in the compressed state. In its non-compressed state, the insert device may preferably obtain a diameter corresponding to the body canal of the subject (in case of the ileum of a person the diameter could be 2-5 cm) and a length of approximately 20-40 cm. The absorbent material may therefore be configured such that the diameter perpendicular to the longitudinal axis increases by at least 100% from the compressed state to the non-compressed state. Further, the absorbent material **is** configured such that the longitudinal axis (the length of the absorbent material) is increased by at least 100% from the compressed state to the non-compressed state. Additionally, the absorbent material may be configured to expand such that the overall volume of the absorbent material in the non-compressed state is at least factor 4, such as a factor 8, larger than the volume of the absorbent material in the compressed state. The compressed state of the insert device may be realised in a number of different ways, e.g. by the device comprising one or more means for compressing the absorbent material: the absorbent material may be compressed and held in the compressed state either by itself or by a coating and/or an insertion sleeve enclosing the absorbent material. The coating is preferably water-soluble and configured to dissolve upon exposure to the moisture, intestinal fluids, and/or heat levels inside the subject. Accordingly, the insert device may comprise a coating and/or an insertion sleeve enclosing the absorbent material. As an example, the absorbent material may be compressed and wrapped in a polymer film, such as a water-soluble synthetic polymer film, the polymer film thereby constituting the coating. An example of a suitable polymer film is a poly(vinyl alcohol) (PVAI) film. However, other coatings may be used. Alternatively, the device is compressed in an applicator and inserted and released inside the body canal, e.g. the small intestine. The applicator material may be paper, cardboard or plastic. Another insertion method consists of an insertion probe in terms of a catheter-like thin tube that is placed inside the absorbent insert device and guides the absorbent insert device into place whereby the probe is pulled out. Suited materials for a probe/guiding catheter are latex, silicone, rubber, plastic or nitinol. Upon placement of the absorbent insert device inside the body canal, e.g. ileum, of the subject, the insert device is configured to expand to a size sufficient to seal the body canal, once the water-soluble coating is dissolved and/or the insertion sleeve is removed. The insert device preferably expands both in the radial and longitudinal direction.

The absorbent insert device comprises an expandable elongated body. The elongated body may comprise or be made entirely from an absorbent material. Alternatively, the elongated body may form a receptacle, such as a tube, filled or partly filled with the absorbent material. The absorbent material may be provided as a woven fabric, a nonwoven fabric, or a foam. An example of a nonwoven fabric is an air-laid paper. The absorbent material may be selected from the group of: carboxymethyl cellulose (CMC), alginate, super absorbent polymers (SAP), super absorbent fibers, plant fibers/polymers, and/or combinations thereof. In case the absorbent material is provided as a foam, the foam may be selected from the group of: polyurethane foam, silicone foam, polyvinyl chloride (PVC) foam, hydrophilic polymer foam, foam rubber, a polymer foam having closed cells or other hydrophilic polymers.

The absorbent insert device preferably comprises withdrawal means attached to the elongated body, e.g. at one end of said body, such that the device can be withdrawn with ease from the subject using said withdrawal means. The withdrawal means may be a string or a cord such that the insert device may be removed from the subject by pulling the string/cord. As an example, the string or cord may be made of cotton, nylon, silicone, rubber, polyester, and/or combinations thereof. However, other materials for the string/cord could be used. Preferably, the withdrawal means is configured to protrude from the stoma, when the insert device is placed inside the body canal, such that the subject can pull the withdrawal means through the stoma to remove the insert device from the body canal. Any of the embodiments of the insert device as described herein may be provided with the withdrawal means. To ensure the absorbent device stays in place, a button or similar may be attached to the end of the withdrawal means.

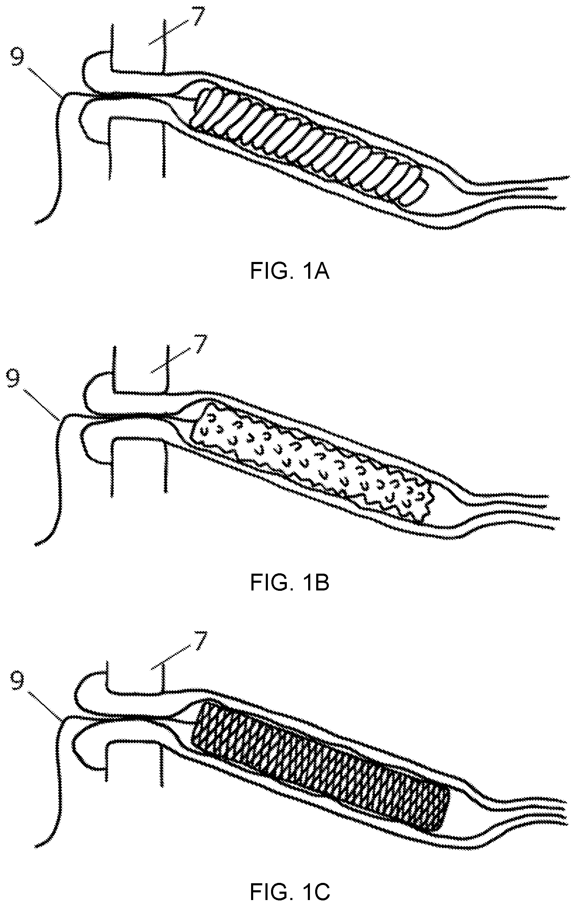

The absorbent insert device may be provided in many different shapes and sizes. According to one embodiment, the elongated body of the insert device is curled around a longitudinal axis of the body to form a helix comprising a plurality of turns. This embodiment is shown both in fig. 1A (compressed state) and in fig. 2A (non-compressed state). The helix can be described by two diameters. A first diameter corresponding to the diameter of the (non-curled) elongated body, and a second diameter corresponding to the outer diameter of the helix, i.e. the diameter of each turn of the helix. The first diameter should preferably be equal to or less than the diameter of the stoma of the subject, such that the insert device can be withdrawn from the body canal of the subject without squeezing the elongated body, since this would likely result in some of the absorbed intestinal waste being discharged from the absorbent material. Hence, by choosing a first diameter of approximately the size of the stoma of interest, the absorbent insert device can ideally be withdrawn from the subject without spillage of intestinal waste inside the body canal. The second diameter associated with the helix preferably ensures that the absorbent insert device conforms to the diameter of the body canal, such that the insert device can form a closure of said canal. The elongated body may comprise a support structure configured to increase the axial stability of the body. The support structure may comprise a material selected from the group of: silicone, rubber, latex, polyethylene, polyvinyl chloride, nitinol, and/or combinations thereof. The support structure may be provided in the centre of the strand forming the helix.

According to another embodiment, the elongated body of the insert device comprises a plurality of flexible protrusions extending radially from the body, such that the elongated body resembles a brush. This embodiment is shown in fig. 1B and fig. 2B. The protrusions of the body are preferably fixated on a core that is either solid or tubular. The flexible protrusions are configured to bend towards the core such that the insert device can be pulled out through the stoma with a minimum of squeezing of the absorbent material. Hence, the elongated body of this embodiment may be described by two diameters: a first diameter that corresponds roughly to the diameter of the stoma (where the protrusions are collapsed along the core) and a second diameter that corresponds roughly to the diameter of the body canal of interest, where the device is intended to be placed, cf. fig. 2B. The second diameter corresponds to the case, where the protrusions extend from the core without being collapsed (when placed inside the body canal of the subject). Therefore, the second diameter is larger than the first diameter. In order to extract the insert device more easily through the stoma of the subject (e.g. an intestinal opening with a diameter of approximately 1 cm), the core of the body should preferably be elastic deformable, such that the device is able to contract radially upon extraction. A plastic or elastic deformable core could be manufactured in e.g. silicone, rubber, latex, polyethylene, polyvinyl chloride, and/or combinations thereof. The core could also be provided as a woven crinoline tube, for example comprising nylon, polyester, PVDF, polypropylene, and/or combinations thereof.

According to yet another embodiment, the elongated body of the insert device is provided as a flexible tube, which is preferably partly or entirely filled with the absorbent material. Alternatively, the flexible tube could be blended or coated with the absorbent material. As an example, the flexible tube may be a woven crinoline tube comprising a material selected from the group of: nylon, polyester, polyvinylidene fluoride (PVDF) or polypropylene. Alternatively, the crinoline tube can be woven (partly or entirely) in the absorbent material, examples of which are mentioned elsewhere herein. The crinoline tube could be similar in appearance to a coronary stent. This embodiment is shown in fig. 1C and fig. 2C. The flexible tube should preferably be configured to contract radially when pulled out through the stoma of the subject, cf. fig. 2C.

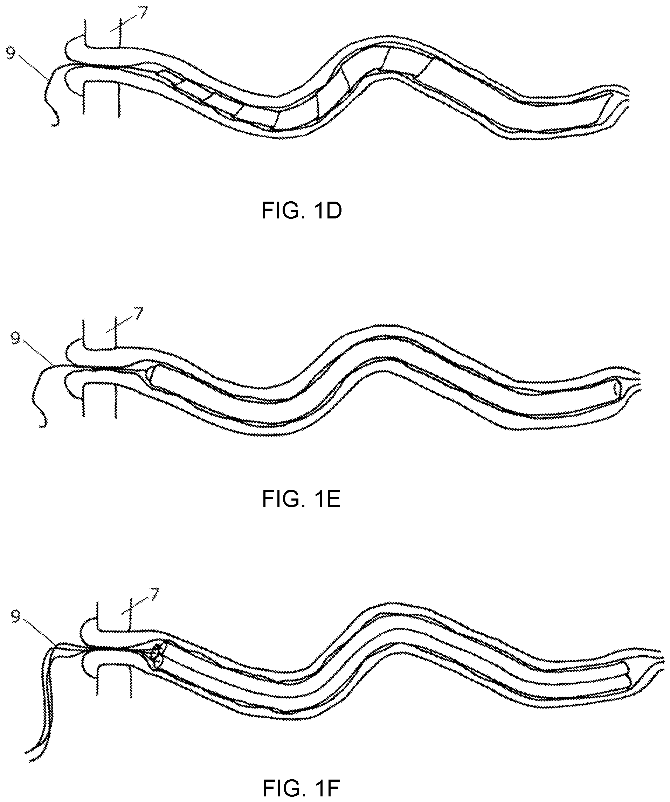

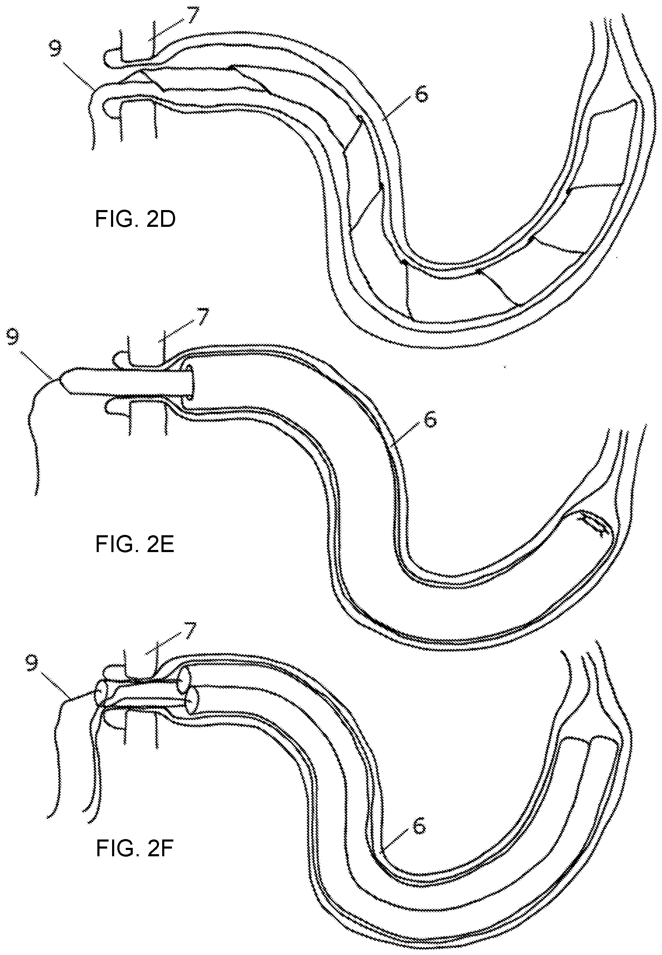

According to yet another embodiment, the elongated body of the insert device is formed from a sheet or strip, which is rolled into an elongated conical shape. This embodiment is shown in fig. 1D and fig. 2D. Preferably, the elongated body is formed from a rectangular sheet of absorbent material (e.g. 10 x 30 cm) that is rolled a bit skewed into a conus shape. The absorbent material may be chosen among the varieties described elsewhere in the application. The elongated body of this embodiment is provided with withdrawal means, such that the insert device may be withdrawn from the subject. The rolled sheet ensures that the device may be withdrawn through a body opening, which is smaller in diameter than the body canal, wherein the device is places. Preferably, the rolled sheet is configured to unfold upon extraction of the device, whereby the sheet can be pulled out in one layer like a spiral. Similar to the other embodiments, this design preferably ensures that a minimum of intestinal waste is squeezed out of the absorbent material upon extraction.

According to yet another embodiment, the elongated body of the insert device is formed from a tube having a closed end, said tube being folded one or more times inside itself, wherein the tube is configured to unfold upon pulling the device out of the stoma by use of the withdrawal means. This embodiment is shown in fig. 1E and fig. 2E. The folded design according to this embodiment ensures that the insert device has a larger diameter inside the body canal, such that it approximately conforms to the body canal and forms a closure thereof. The ability of the tube to unfold ensures that the device may be withdrawn from the body canal out through the stoma (e.g. an intestinal opening of diameter approximately 1 cm), wherein said stoma has a diameter less than the diameter of the body canal. The tube is preferably folded one or two times inside itself, to obtain a length of ½ or 1/3 of the initial length. As an example, the tube may initially be approximately 60 cm in length before being folded. The elongated body of this embodiment is provided with withdrawal means as described elsewhere. The tube may comprise or consist of the absorbent material according to any of the varieties mentioned elsewhere. The tube may be provided with longitudinal seams or strings to enhance the stiffness of the tube. These seams or strings may be made of nylon, polyester, PVDF, polypropylene, silicone, rubber, latex and/or combinations thereof, or other suitable materials for this purpose.

According to yet another embodiment, the elongated body of the insert device is formed from a plurality of cylinders, each cylinder comprising an absorbent material. This embodiment is shown in fig. 1F and fig. 2F. Preferably, each cylinder is provided with withdrawal means, such as a string, attached e.g. to an end of each cylinder, such that the plurality of cylinders may be withdrawn from the subject individually. Preferably, each cylinder has a diameter less than or equal to the diameter of the stoma of interest. This ensures that each cylinder may be withdrawn through the stoma without being squeezed by the body opening. The plurality of cylinders ensures that the diameter of the elongated body (i.e. here the outer diameter of the combined cylinders), approximately conforms to the body canal in order to seal the canal. This embodiment may be provided with a support structure configured to increase the axial stability of the body. As an example, the support structure can comprise silicone, rubber, latex, polyethylene, polyvinyl chloride, and/or combinations thereof. Alternatively, one or more of the plurality of cylinders may be provided with longitudinal seams or strings in nylon, polyester, PVDF or polypropylene.

Any of the described embodiments of the absorbent insert device may further comprise a sensor, which is preferably configured to detect if the absorbent insert device has been wetted (e.g. due to absorption of liquid) and/or configured to provide microbial agents or biochemical information (such as pH level, level of electrolytes, and/or presence of blood). The sensor may be attached to the absorbent insert device or it may be embedded in the insert device.

Any of the described embodiments may further comprise a support structure to increase the stiffness of the elongated body of the insert device. Said support structure preferably comprises a material selected from the group of: silicone, rubber, latex, polyethylene, polyvinyl chloride, nitinol, and/or combinations thereof. Any of the described embodiments may further comprise a removable insertion tube configured to aid in inserting the device through the stoma of the subject, said insertion tube being configured to be withdrawn after the insert device has been placed in the body canal of the subject. Any of the described embodiments may further comprise a water-soluble coating enclosing the elongated body, said coating being configured to dissolve upon exposure to the moisture and/or heat levels inside the subject. All of the described embodiments have in common that they are configured to be able to be withdrawn from the subject while minimizing squeezing of the device at the opening, and thereby minimizing spillage of intestinal waste inside the body canal. This ability is achieved by the different design/shapes as described in relation to the different embodiments. Preferably, all of the embodiments of the insert device are configured to have a predefined size and shape to avoid a too high pressure on the wall of the body canal of the subject, wherein the insert device is intended to be placed.

The described embodiments may be combined to form new embodiments. As an example, the embodiment resembling a brush (fig. 1 B and 2B) may be turned to form a helix as described in relation to the embodiment of fig. 1A and fig. 2A. Another example is the flexible tube described in relation to fig. 1C, which may be used in replacement of one or more of the cylinders described in relation to fig. 1F. Other embodiments can be made from other combinations of the disclosed embodiments.

The initial length of the elongated body of the insert device corresponds to a compressed state of the elongated body. The compressed length of the elongated body is preferably less than 20 cm, even more preferably less than 15 cm. In the non-compressed state of the elongated body, said body **has an** expanded length of at least 15 cm, such as 15-50 cm.

Accordingly, the elongated body is preferably configured to expand to a length of more than 15 cm, preferably more than 20 cm, when placed in the body canal of the subject. In the expanded state of the insert device, the elongated body preferably has a diameter approximately similar to the diameter of the body canal, wherein it is placed. In some applications, this corresponds to a diameter of approximately 2-3 cm. Preferably, the insert device has an absorption capacity of at least 50 millilitres, preferably at least 100 millilitres, more preferably at least 150 millilitres, even more preferably at least 200 millilitres or even up to 500 millilitres. The insert device is preferably configured to retain at least 75 % of the absorbed intestinal waste upon pulling the device out through the stoma, e.g. ileostomy, of the subject. Even more preferably, the insert device is configured to retain at least 90 % of the absorbed intestinal waste when the device is withdrawn from the subject.

### Detailed description of the drawings

Fig. 1A shows the absorbent insert device according to one embodiment of the present disclosure. This figure illustrates the insert device when it is in a compressed state and when it is placed in the body canal of a subject. This embodiment of the insert device comprises an elongated body, which is curled around a longitudinal axis of the body to form a helix comprising a plurality of turns.
Fig. 1B shows the absorbent insert device according to another embodiment of the present disclosure. This figure illustrates the insert device when it is in a compressed state and when it is placed in the body canal of a subject. This embodiment of the insert device comprises an elongated body with a plurality of flexible protrusions extending radially from the body, such that the elongated body resembles a brush.
Fig. 1C shows the absorbent insert device according to another embodiment of the present disclosure. This figure illustrates the insert device when it is in a compressed state and when it is placed in the body canal of a subject. In this embodiment, the elongated body of the insert device is provided as a flexible tube, which is preferably partly or entirely filled with the absorbent material.
Fig. 1D shows the absorbent insert device according to another embodiment of the present disclosure. This figure illustrates the insert device when it is in a compressed state and when it is placed in the body canal of a subject. In this embodiment, the elongated body of the insert device is formed from a sheet, which is rolled into an elongated conical shape.
Fig. 1E shows the absorbent insert device according to another embodiment of the present disclosure. This figure illustrates the insert device when it is in a compressed state and when it is placed in the body canal of a subject. In this embodiment, the elongated body of the insert device is formed from a tube having a closed end, said tube being folded one or more times inside itself, wherein the tube is configured to unfold upon pulling the device out of the stoma by use of the withdrawal means.
Fig. 1F shows the absorbent insert device according to another embodiment of the present disclosure. This figure illustrates the insert device when it is in a compressed state and when it is placed in the body canal of a subject. In this embodiment, the elongated body of the insert device is formed from a plurality of cylinders, each cylinder comprising an absorbent material.
Figures 2A-2F shows a non-compressed state of the insert device according to the corresponding (compressed) embodiment of fig. 1A-1 F. Hence, fig. 2A is the non-compressed (expanded) state of the embodiment shown in fig. 1A and so forth. For each of the embodiments, the insert device is preferably configured to expand to form a seal of the body canal, wherein it is placed, once the compression (e.g. provided by a coating or an insertion-sleeve) is removed. Further, it can be seen on all the figures, that the devices is shaped/configured such the diameter of the absorbent material at the cross-section of the stoma, upon removal, is substantially similar to the diameter of the stoma. This diameter is different from the diameter of the absorbent material as expanded in the non-compressed state.

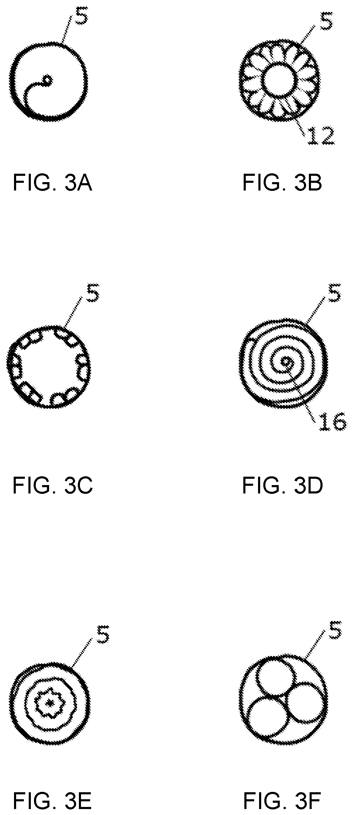

Figures 3A-3F shows cross-sections of the embodiments shown in figures 1A-1F. These cross-sections correspond to the compressed state of the insert device.

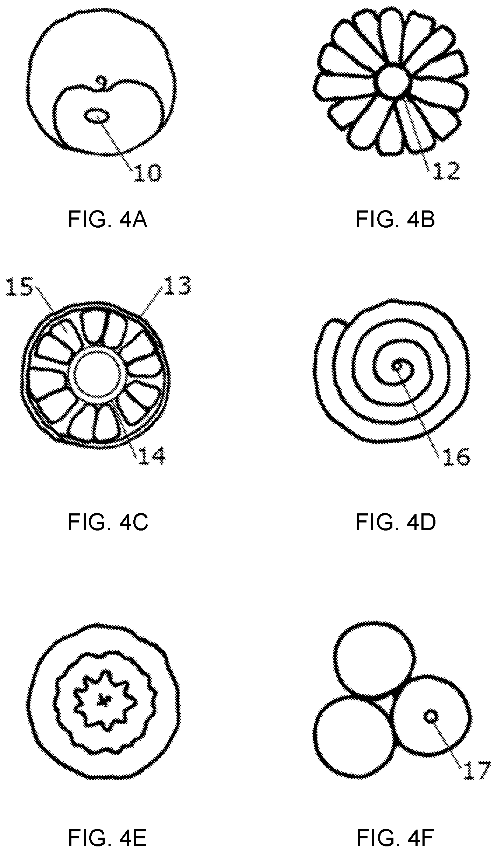

Figures 4A-4F shows cross-sections of the embodiments shown in figures 2A-2F. These cross-sections correspond to the non-compressed state of the insert device.

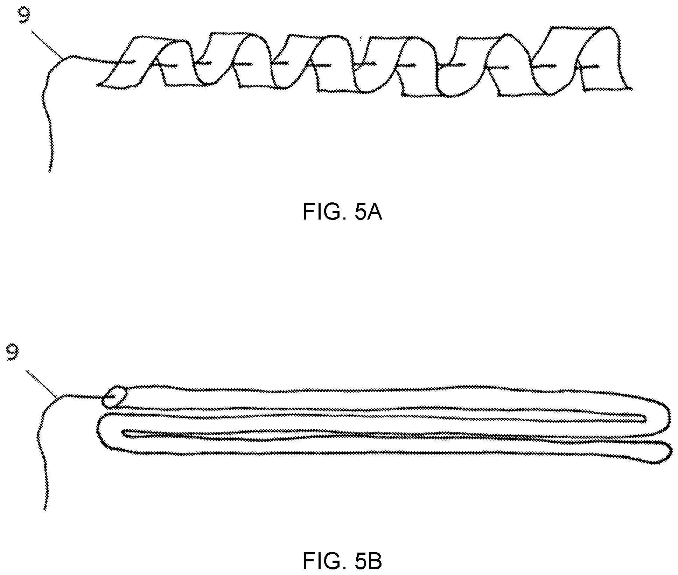

Fig. 5A shows the absorbent insert device according to another embodiment of the present disclosure. This embodiment of the insert device comprises an elongated body, which is formed from a sheet that is folded to form a wave-like pattern of crests and troughs.
Fig. 5B shows the absorbent insert device according to another embodiment of the present disclosure. This embodiment of the insert device comprises an elongated body constituting a cylinder that is folded one or more times.

### Reference list

5 - Compressed state
6 - Body canal
7 - Stoma
9 - Withdrawal means
10, 12, 14, 16, 17 - Attachment to withdrawal means
13 - Non-compressed state
15 - Brush (non-compressed)

## Claims

1. An absorbent insert ileostomy device for insertion through an ileostomy of a human being, the device being configured to absorb intestinal waste inside the ileum of the human being and to form a leak-tight closure of the ileostomy, the device comprising:
- an expandable elongated body comprising an absorbent material,
- withdrawal means (9) attached to the elongated body, and
- one or more compression means;
wherein the compression means is configured to maintain the expandable elongated body in a compressed state (5), having a first diameter, prior to insertion;
wherein the expandable elongated body is configured to expand radially to a non-compressed state (13), having a second diameter, when released from the compression means upon insertion into the ileum through the ileostomy;
wherein the first diameter is equal to or less than the diameter of the ileostomy of the human being and the second diameter is equal to or larger than the diameter of the ileum of the human being;
wherein the insert device has a diameter of 1-3 cm and a length of 5-20 cm in the compressed state (5), and wherein the second diameter is between 2 and 5 cm, **characterized in that** the absorbent material may be configured such that the length of the absorbent material is increased by at least 100% from the compressed state to the non-compressed state,
wherein the length of the expandable elongated body in the non-compressed state (13) along the longitudinal axis is at least 15 cm.

2. The absorbent insert device according to claim 1, wherein the elongated body comprising the absorbent material is configured to conform to a third diameter when pulled out of the ileum of the human being by means of the withdrawal means (9); wherein the third diameter is the diameter of the ileostomy.

3. The absorbent insert device according to any of the preceding claims, wherein the absorbent insert device has an absorption capacity of at least 50 millilitres.

4. The absorbent insert device according to any of the preceding claims, wherein the elongated body is curled around a longitudinal axis of the body to form a helix comprising a plurality of turns.

5. The absorbent insert device according to any of the preceding claims, wherein the elongated body comprises a plurality of flexible protrusions extending radially from the body, such that the elongated body resembles a brush (15).

6. The absorbent insert device according to any of the preceding claims, wherein the elongated body is provided as a flexible tube, which is partly or entirely filled with the absorbent material.

7. The absorbent insert device according to claim 6, wherein the flexible tube is a woven crinoline tube comprising a material selected from the group of: nylon, polyester, polyvinylidene fluoride (PVDF) or polypropylene.

8. The absorbent insert device according to any of the preceding claims, wherein the elongated body is formed from a sheet, which is rolled into an elongated conical shape.

9. The absorbent insert device according to any of the preceding claims, wherein the elongated body is formed from a tube having a closed end, said tube being folded one or more times inside itself, wherein the tube is configured to unfold upon pulling the device out of the ileostomy by use of the withdrawal means (9).

10. The absorbent insert device according to any of the preceding claims, wherein the elongated body comprises a plurality of cylinders, each cylinder comprising an absorbent material, and wherein withdrawal means (9) is attached to each cylinder, such that the plurality of cylinders may be withdrawn from the subject individually.

11. The absorbent insert device according to any of the preceding claims, wherein the elongated body comprises a support structure configured to increase the axial stability of the body, wherein said support structure comprises a material selected from the group of: silicone, rubber, latex, polyethylene, polyvinyl chloride, nitinol, and/or combinations thereof.

12. The absorbent insert device according to any of the preceding claims, wherein the length in the non-compressed state (13) is 20-40 cm.

13. The absorbent insert device according to any of the preceding claims, wherein the absorbent material is configured such that the diameter perpendicular to the longitudinal axis increases by at least 100% from the compressed state to the non-compressed state

14. The absorbent insert device according to any of the preceding claims, wherein the absorbent material is configured to expand such that the overall volume of the absorbent material in the non-compressed state is at least factor 4 larger than the volume of the absorbent material in the compressed state.

## Patentansprüche

1. Saugfähige Enterostoma-Einsetzvorrichtung zum Einsetzen durch ein Enterostoma eines Menschen, wobei die Vorrichtung dazu konfiguriert ist, Darmabfall im Innern des Ileums des Menschen zu absorbieren und einen dichten Abschluss des Enterostomas zu bilden, wobei die Vorrichtung Folgendes umfasst:
- einen dehnbaren länglichen Körper, der ein saugfähiges Material umfasst,
- Entnahmemittel (9), die an dem länglichen Körper befestigt sind, und
- ein oder mehrere Kompressionsmittel;
wobei das Kompressionsmittel dazu konfiguriert ist, den dehnbaren länglichen Körper in einem komprimierten Zustand (5) zu halten, der vor dem Einsetzen einen ersten Durchmesser aufweist;
wobei der dehnbare längliche Körper dazu konfiguriert ist, sich radial in einen nicht komprimierten Zustand (13), der einen zweiten Durchmesser aufweist, zu dehnen, wenn er von dem Kompressionsmittel beim Einsetzen in den Ileus durch das Enterostoma freigegeben wird;
wobei der erste Durchmesser kleiner oder gleich dem Durchmesser des Enterostomas des Menschen ist und der zweite Durchmesser größer oder gleich dem Durchmesser des Ileus des Menschen ist;
wobei die Einsetzvorrichtung einen Durchmesser von 1-3 cm und eine Länge von 5-20 cm in dem komprimierten Zustand (5) aufweist und wobei der zweite Durchmesser zwischen 2 und 5 cm beträgt, **dadurch gekennzeichnet, dass** das saugfähige Material derart konfiguriert sein kann, dass sich die Länge des saugfähigen Materials von dem komprimierten Zustand in den nicht komprimierten Zustand um mindestens 100 % erhöht, wobei die Länge des dehnbaren länglichen Körpers in dem nicht komprimierten Zustand (13) entlang der Längsachse mindestens 15 cm beträgt.

2. Saugfähige Einsetzvorrichtung nach Anspruch 1, wobei der längliche Körper, der das saugfähige Material umfasst, dazu konfiguriert ist, sich an einen dritten Durchmesser anzupassen, wenn er mithilfe der Entnahmemittel (9) aus dem Ileus des Menschen gezogen wird; wobei der dritte Durchmesser der Durchmesser des Enterostomas ist.

3. Saugfähige Einsetzvorrichtung nach einem der vorhergehenden Ansprüche, wobei die saugfähige Einsetzvorrichtung eine Saugfähigkeit von mindestens 50 Millilitern aufweist.

4. Saugfähige Einsetzvorrichtung nach einem der vorhergehenden Ansprüche, wobei der längliche Körper um eine Längsachse des Körpers gewunden ist, um eine Helix zu bilden, die eine Vielzahl von Windungen umfasst.

5. Saugfähige Einsetzvorrichtung nach einem der vorhergehenden Ansprüche, wobei der längliche Körper eine Vielzahl flexibler Vorsprünge umfasst, die sich derart radial von dem Körper erstrecken, dass der längliche Körper einer Bürste (15) ähnelt.

6. Saugfähige Einsetzvorrichtung nach einem der vorhergehenden Ansprüche, wobei der längliche Körper als ein flexibler Schlauch bereitgestellt ist, der teilweise oder ganz mit dem saugfähigen Material gefüllt ist.

7. Saugfähige Einsetzvorrichtung nach Anspruch 6, wobei der flexible Schlauch ein gewebter Krinolinen-Schlauch ist, der ein Material umfasst, das aus der folgenden Gruppe ausgewählt ist: Nylon, Polyester, Polyvinylidenfluorid (PVDF) oder Polypropylen.

8. Saugfähige Einsetzvorrichtung nach einem der vorhergehenden Ansprüche, wobei der längliche Körper von einer Folie gebildet ist, die in eine längliche konische Form gerollt ist.

9. Saugfähige Einsetzvorrichtung nach einem der vorhergehenden Ansprüche, wobei der längliche Körper von einem Schlauch gebildet ist, der ein geschlossenes Ende aufweist, wobei der Schlauch ein- oder mehrmals in sich selbst gefaltet ist, wobei der Schlauch dazu konfiguriert ist, sich beim Herausziehen der Vorrichtung aus dem Enterostoma unter Verwendung der Entnahmemittel (9) zu entfalten.

10. Saugfähige Einsetzvorrichtung nach einem der vorhergehenden Ansprüche, wobei der längliche Körper eine Vielzahl von Zylindern umfasst, wobei jeder Zylinder ein saugfähiges Material umfasst, und wobei das Entnahmemittel (9) derart an jedem Zylinder befestigt ist, dass die Vielzahl von Zylindern einzeln aus dem Patienten entnommen werden kann.

11. Saugfähige Einsetzvorrichtung nach einem der vorhergehenden Ansprüche, wobei der längliche Körper eine Stützstruktur umfasst, die dazu konfiguriert ist, die axiale Stabilität des Körpers zu erhöhen, wobei die Stützstruktur ein Material umfasst, das aus der folgenden Gruppe ausgewählt ist: Silikon, Gummi, Latex, Polyethylen, Polyvinylchlorid, Nitinol und/oder Kombinationen davon.

12. Saugfähige Einsetzvorrichtung nach einem der vorhergehenden Ansprüche, wobei die Länge in dem nicht komprimierten Zustand (13) 20-40 cm beträgt.

13. Saugfähige Einsetzvorrichtung nach einem der vorhergehenden Ansprüche, wobei das saugfähige Material derart konfiguriert ist, dass sich der Durchmesser senkrecht zu der Längsachse von dem komprimierten in den nicht komprimierten Zustand um mindestens 100 % erhöht.

14. Saugfähige Einsetzvorrichtung nach einem der vorhergehenden Ansprüche, wobei das saugfähige Material dazu konfiguriert ist, sich derart auszudehnen, dass das Gesamtvolumen des saugfähigen Materials in dem nicht komprimierten Zustand mindestens um den Faktor 4 größer ist als das Volumen des saugfähigen Materials in dem komprimierten Zustand.

## Revendications

1. Dispositif d'insert absorbant d'iléostomie pour l'insertion à travers une iléostomie d'un être humain, le dispositif étant configuré pour l'absorption de déchets intestinaux à l'intérieur de l'iléon de l'être humain et pour la formation d'une fermeture étanche aux fuites de l'iléostomie, le dispositif comprenant :
- un corps allongé expansible comprenant un matériau absorbant,
- des moyens de retrait (9) fixés au corps allongé, et
- un ou plusieurs moyens de compression ;
dans lequel le moyen de compression est configuré pour le maintien du corps allongé expansible dans un état comprimé (5), ayant un premier diamètre, avant l'insertion ;
dans lequel le corps allongé expansible est configuré pour l'expansion radiale vers un état non comprimé (13), ayant un second diamètre, lorsqu'il est libéré du moyen de compression lors de l'insertion à l'intérieur de l'iléon à travers l'iléostomie ;
dans lequel le premier diamètre est égal ou inférieur au diamètre de l'iléostomie de l'être humain et le second diamètre est égal ou supérieur au diamètre de l'iléon de l'être humain ;
dans lequel le dispositif d'insert a un diamètre de 1 à 3 cm et une longueur de 5 à 20 cm dans l'état comprimé (5), et dans lequel le second diamètre est compris entre 2 et 5 cm, **caractérisé en ce que** le matériau absorbant peut être configuré de telle sorte que la longueur du matériau absorbant est augmentée d'au moins 100 % à partir de l'état comprimé jusqu'à l'état non comprimé,
dans lequel la longueur du corps allongé expansible dans l'état non comprimé (13) le long de l'axe longitudinal est d'au moins 15 cm.

2. Dispositif d'insert absorbant selon la revendication 1, dans lequel le corps allongé comprenant le matériau absorbant est configuré pour la conformation à un troisième diamètre lorsqu'il est tiré hors de l'iléon de l'être humain au moyen des moyens de retrait (9) ; dans lequel le troisième diamètre est le diamètre de l'iléostomie.

3. Dispositif d'insert absorbant selon l'une quelconque des revendications précédentes, dans lequel le dispositif d'insert absorbant a une capacité d'absorption d'au moins 50 millilitres.

4. Dispositif d'insert absorbant selon l'une quelconque des revendications précédentes, dans lequel le corps allongé est enroulé autour d'un axe longitudinal du corps pour la formation d'une hélice comprenant une pluralité de spires.

5. Dispositif d'insert absorbant selon l'une quelconque des revendications précédentes, dans lequel le corps allongé comprend une pluralité de saillies flexibles s'étendant radialement à partir du corps, de sorte que le corps allongé ressemble à une brosse (15).

6. Dispositif d'insert absorbant selon l'une quelconque des revendications précédentes, dans lequel le corps allongé est fourni en tant que tube flexible, lequel est partiellement ou entièrement rempli avec le matériau absorbant.

7. Dispositif d'insert absorbant selon la revendication 6, dans lequel le tube flexible est un tube de crinoline tissé comprenant un matériau sélectionné dans le groupe constitué de : nylon, polyester, polyfluorure de vinylidène (PVDF) ou polypropylène.

8. Dispositif d'insert absorbant selon l'une quelconque des revendications précédentes, dans lequel le corps allongé est formé à partir d'une feuille, laquelle est roulée en une forme conique allongée.

9. Dispositif d'insert absorbant selon l'une quelconque des revendications précédentes, dans lequel le corps allongé est formé à partir d'un tube ayant une extrémité fermée, ledit tube étant plié une ou plusieurs fois à l'intérieur de lui-même, dans lequel le tube est configuré pour le dépliage lors de la traction du dispositif hors de l'iléostomie par l'utilisation des moyens de retrait (9).

10. Dispositif d'insert absorbant selon l'une quelconque des revendications précédentes, dans lequel le corps allongé comprend une pluralité de cylindres, chaque cylindre comprenant un matériau absorbant, et dans lequel un moyen de retrait (9) est fixé à chaque cylindre, de sorte que la pluralité de cylindres peuvent être retirés à partir du sujet individuellement.

11. Dispositif d'insert absorbant selon l'une quelconque des revendications précédentes, dans lequel le corps allongé comprend une structure de support configurée pour l'augmentation de la stabilité axiale du corps, dans lequel ladite structure de support comprend un matériau sélectionné dans le groupe constitué de : le silicone, le caoutchouc, le latex, le polyéthylène, le chlorure de polyvinyle, le nitinol et/ou des combinaisons de ceux-ci.

12. Dispositif d'insert absorbant selon l'une quelconque des revendications précédentes, dans lequel la longueur dans l'état non comprimé (13) est de 20 à 40 cm.

13. Dispositif d'insert absorbant selon l'une quelconque des revendications précédentes, dans lequel le matériau absorbant est configuré de sorte que le diamètre perpendiculaire à l'axe longitudinal augmente d'au moins 100 % à partir de l'état comprimé jusqu'à l'état non comprimé.

14. Dispositif d'insert absorbant selon l'une quelconque des revendications précédentes, dans lequel le matériau absorbant est configuré pour l'expansion de sorte que le volume global du matériau absorbant dans l'état non comprimé est au moins un facteur 4 plus grand que le volume du matériau absorbant dans l'état comprimé.
